# EUROPEAN PATENT SPECIFICATION

(11) **EP 0 995 115 B1**
(45) Date of publication and mention of the grant of the patent: **19.05.2004**
(21) Application number: 97933668.2
(22) Date of filing: 12.07.1997
(51) Int. Cl.: G01N 33/00, G01D 7/02

(54) **DEVICE FOR MONITORING THE AIR QUALITY**
VORRICHTUNG ZUM ÜBERWACHEN DER LUFTQUALITÄT
DISPOSITIF DE SURVEILLANCE DE LA QUALITE DE L'AIR

(43) Date of publication of application: 26.04.2000
(73) Proprietor: IDT Technology Limited, Hong Kong (CN)
(72) Inventor: CHAN, Raymond Apartment E10, Hong Kong (CN)
(74) Representative: Lusuardi, Werther Giovanni, Dr.
(86) International application number: PCT/EP1997/003732
(87) International publication number: WO 1999/002987

(56) References cited:
- EP-A- 0 513 420
- WO-A-95/04957
- DE-U- 9 217 465
- DE-U- 29 601 472
- US-A- 4 562 723

## Description

This invention concerns a device in accordance with Claim 1.

Different types of air quality monitors are already known which, however, are measuring and displaying only single, momentaneous values which do not allow to predict trends or to facilitate decision to be taken for maintaining safety of or for restoring comfort of indoor conditions.

From the DE C-2 857 262 a conventional gas alerting device is known using various gas sensors and which is able to produce a warning signal when certain values in gas concentration are surpassed.

From the DE 296 01 472 an apparatus according to the preamble of claim 1 is known. This known apparatus has two disadvantages, the first being that the measurement of the gas value occurs by manually actuating a switch and cannot be performed automatically; the second being the lack of a simultaneous indication of the current and previous measured values.

From EP-A-0 513 420 and US-A-4 562 723 further apparatus according to the preamble of claim 1 are known with similar disadvantages.

The invention as claimed aims at solving the above described problems with state of the art devices and to allow indication of air quality history which allows the user to take appropriate measures for improving air quality or to avoid uncomfortable or dangerous situations.

The present invention provides a device for monitoring the air quality as defined in Claim 1.

The various features of novelty which characterize the invention are pointed out with particularity in the claims annexed to and forming part of this disclosure. For the better understanding of the invention, its operating advantages and specific objects attained by its use, reference should be had to the accompanying drawings, examples and descriptive matter in which are illustrated and described preferred embodiments of the invention.

In the drawings:
Fig. 1 shows a block circuit diagram of a device for monitoring the air quality according to the invention;
Fig. 2 shows the front panel of a device for monitoring the air quality;
Fig. 3 shows a side view of a device for monitoring the air quality;
Fig. 4 shows the indicating arrangements of a device monitoring the air quality (full segment);
Fig. 5 shows the indicating arrangements of a device monitoring the air quality in the working mode indicating a very poor condition of air quality;
Fig. 6 shows the indicating arrangements of a device monitoring the air quality in the working mode indicating a good condition of air quality;
Fig. 7 shows a bottom view of a device for monitoring the air quality;
Fig. 8 shows a top view of a device for monitoring the air quality; and
Fig. 9 shows a rear view of a device for monitoring the air quality.

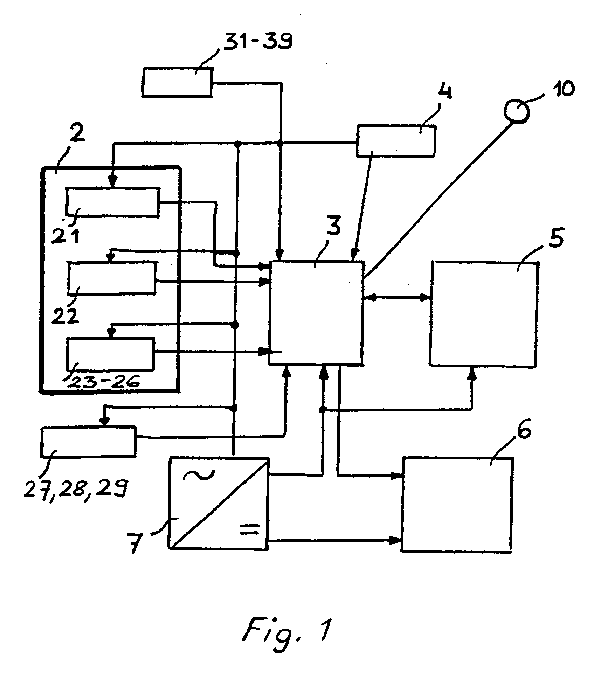

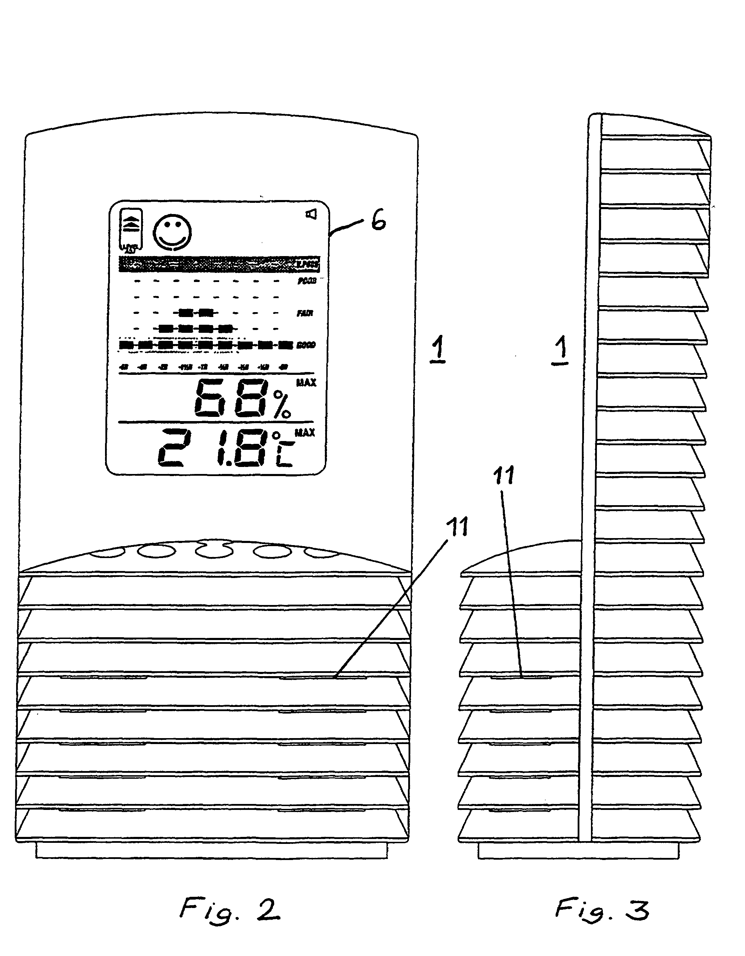

The device 1 for monitoring the air quality is illustrated in Figs. 2, 3, 7 - 9. The function of the device 1 can best be described having reference to the block circuit diagram represented in detail in Figure 1. It contains a sensor arrangement 2 for measuring a variety of chemical gases and of meteorological parameters which play an influential part in perception of air quality comfort. Said arrangement 2 comprises at least one of the following gas sensors:
sensor 21 for measuring volatile organic compounds VOC;
sensor 22 for measuring carbon dioxide CO₂;
sensor 23 for measuring nitric oxides NOₓ;
sensor 24 for measuring carbon monoxide CO;
sensor 25 for measuring reducing gases;
sensor 26 for measuring oxidizing gases.

Gas sensors of the above type are commercially available and well known in the art. Gas sensors for reducing and oxidizing gases are based on reversible conductivity change of the sensing elements and are able to detect a variety of gases. Sensors with SnO₂ sensitive layers are able to detect most reducing gases, e.g. combustible gases (hydrogen, methane, butane a.s.o.), toxic gases (CO, NOₓ), alcoholic and cetonic vapours. Sensors with Nb₂O₅ sensitive layers are able to detect most oxidizing gases, e.g. oxygen and NO₂.
Sensors for VOC gases are based on thermal conductivity. VOC gases (volatile organic compounds) may comprise butane, methane, ethanolic vapours, smelling gases generated by smoke and other burning processes.
Reference is made to the following prior art documents which describe such sensors in detail: US 5.393.399, EP-A-0 479 731, EP-A-0 291 462.

Additional sensors for meteorological data may be used in the device, such as a temperature sensor 27, a barometric pressure sensor 28 and/or a relative air humidity sensor 29.

The outputs from the sensors 21-29 are fed to a computer 3 which is connected to an electronic clock 4, a memory 5 and indicating arrangements 6. A current supply system 7 supplies all the parts of the device with power.

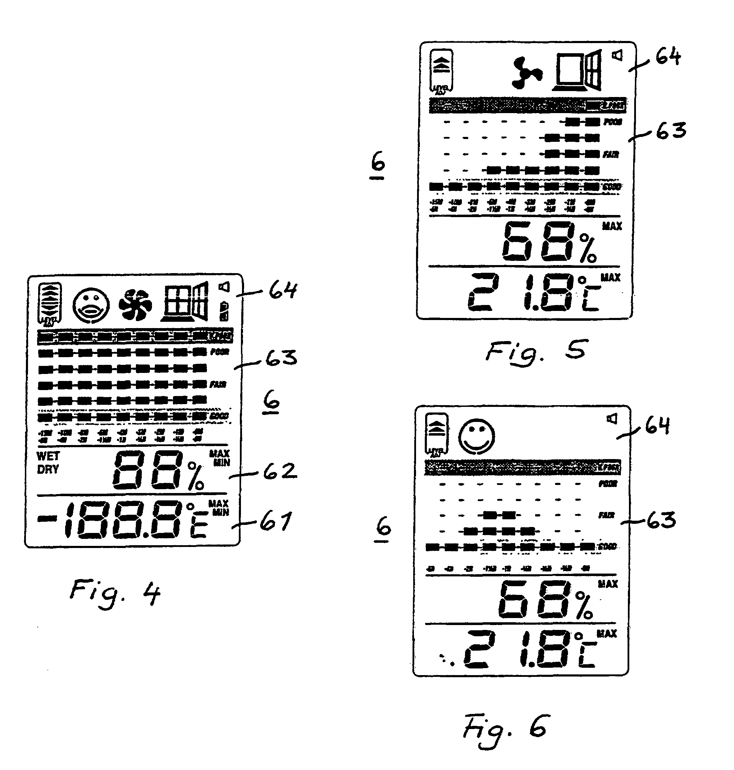

Measurement, amplification, storing, processing, indication and display of the gas concentrations and of the meteorological data as well as storing, indication and display of historical data, as well as derivation and display of trends to be expected are all handled by appropriate software programs. For details of chronological processing of data, reference is made to EP-B-0 513 429 which discloses appropriate procedures for processing barometric pressure data which can be used also for any other type of data.
By means of the device 1 according to the invention it is possible to indicate current measured values and ones which have been measured with the sensor arrangement 2 within a given past period of time at given intervals. It further allows to indicate the associated measured values trends and to display them at the indicating arrangements 6, preferably in digital form, e.g. by means of a bar chart display at the indicating arrangements 6 as shown in Figs. 4 to 6.

According to Fig. 4 the indicating arrangements 6 consist basically of four displays. Display 61 indicates current temperature, display 62 indicates relative humidity, display 63 indicates the air quality in form of a bar chart distinguishing between "very poor", "poor", "fair" and "good" air quality.

Display 64 indicates symbols for proposed or recommended actions/measures to be taken by the user. From left to right the following symbols are available:
- User defined Level Adjustment (arrows)
- Face for three modes :
   - smiling face corresponding to "good"
   - normal face corresponding to "fair"
   - unhappy face for "very poor"
- Fan "turning fan" corresponding to "very poor" air quality
- Window "open window" corresponding to "very poor" air quality

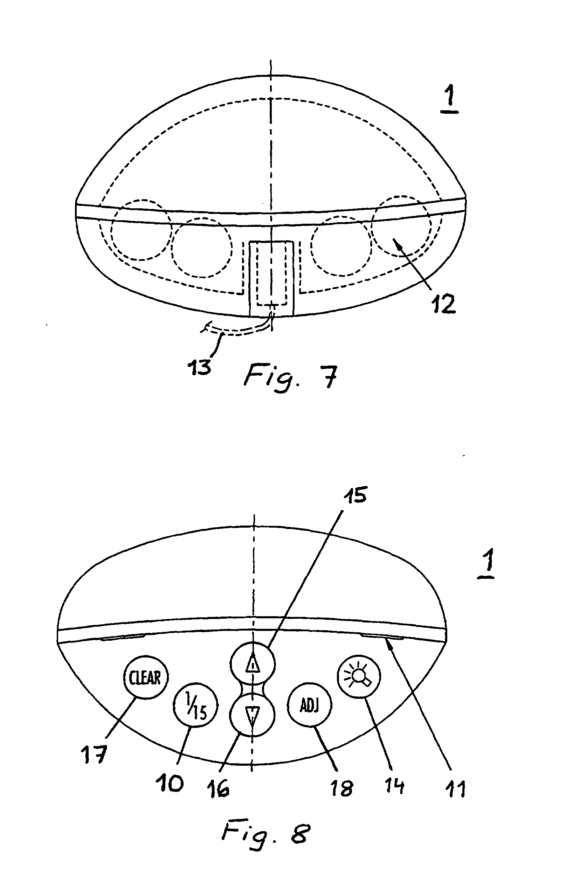

In Fig. 5 a condition of "very poor" air quality is indicated on the indicating arrangements 6. Display 64 shows the fan turning and the window open. The bar chart indicates the actual "very poor" condition in the bar column at the extreme right in display 63. The bar column left from the extreme right column indicates the air condition 1 minute ago, which was "poor". Further left the next column shows the air condition 2 minutes ago which was "fair". The next columns to the left show the air condition 3 minutes, 4 minutes and 6 minutes ago which was still "fair". The next columns to the left show the air condition 8, 10 and 15 minutes ago which was "good".

The above measurement intervals are indicated only as an example. They are, however, small compared with the said period of time (1 to 10 % thereof) and typically in the range of 0,5 to 2,0 minutes, preferably of 0,9 to 1,1 minutes, for measuring short term events.

In Fig. 6 a condition of "good" air quality is indicated on the indicating arrangements 6. Display 64 shows the face smiling only. The bar chart indicates the actual "good" air quality condition in the bar column at the extreme right in display 63. The bar column left from the extreme right column indicates the air condition 1/4 of an hour ago, which was still "good". Further left the next column shows the air condition 1/2 hour ago which was still "good". The next columns to the left show the air condition 3/4, 1, 1,5 and 2 hours ago which was "fair". The next columns to the left show the air condition 4 and 6 hours ago which was "good".

The display 64 in Fig. 6 shows therefore - when the measured values remain constant for a certain time period - that the measurement mode is automatically switched to longer intervals in the range of 10 to 20 minutes, preferably of 14 to 16 minutes, for measuring medium term events. This procedure helps to minimize power consumption which is particularly important when the device is battery operated.
As soon as measured values are significantly changing - or alternatively by acting on an appropriate button 10 (Fig. 8) of the device 1 - the operation mode of the device is again switched to the short term event measurement.

The history bargraphs display in display 64 of the indicating arrangements 6 may comprise the following:
actual values
values 5 minutes ago
values 15 minutes ago
values 30 minutes ago
values 1 hour ago
values 2 hours ago
values 6 hours ago
but can also be arranged in a different pattern.

Part of the computer 3 is set up as an algorithm producer which deduces symbols for the level of air quality from measured values and/or measured values trends fed into the computer 3. Said symbols can be displayed for the level of air quality at the indicating arrangements 6 which are shown in detail in Figs. 4 to 6.

The sensor arrangement 2 may comprise two sensors for each parameter (chemical gas or meteorological data) to be measured, one sensor for indoor measurement and one sensor for outdoor measurement. As an example one may use an indoor sensor 24 for the detection of carbon monoxide CO and an outdoor sensor 34 for the detection of carbon monoxide CO. This double measurement allows to give the user indications about opportunity of opening the window in order to enhance air quality. By appropriate symbols on the indicating arrangements 6 the user may be alerted not to open the window because outdoor conditions are not recommending such an action. The device 1 allows therefore useful indication, at the indicating arrangements 6, whether comfortable and/or safe conditions prevail indoor in the room in which the device 1 is set up, compared to the outdoor conditions.

The device 1 generates by means of algorithms and displays on the indicating arrangements 6 by means of symbols or icons recommended actions to be performed by the user of the device, e.g.
- a symbolic ventilator for recommending ventilation of the room;
- a smiling face for indicating good indoor air quality;
- an open window door for recommending opening the windows.

In a preferred embodiment of the invention the indication of air quality comfort and its numerical or symbolic display on the indicating arrangements 6 can be adjusted to individual perception by lowering or increasing threshold-values triggering changes in the display. This additional feature is of great importance since the human nose is recognized universally to be the best sensor. The device is able by this means to match individual perception of air quality comfort which may vary tremendously from one person to the other ("user accepted level").

The device according to the invention may comprise different combinations of sensors according to the intended use of the device.
A very simple device may consist of an indoor sensor 21 for VOC an indoor temperature sensor 27 and an outdoor temperature sensor 37.

A more sophisticated device may consist of indoor sensors 21 for VOC, 22 for measuring carbon dioxide CO₂, 23 for measuring nitric oxides NOₓ and 27 for measuring indoor temperature, as well as of outdoor sensors 37 for measuring outdoor temperature and 33 for measuring outdoor concentration of nitric oxides NOₓ. For monitoring air quality in cities and in heavy traffic conditions the device 1 may consist of indoor sensors 23 for measuring nitric oxides NOₓ, 24 for carbon monoxide and 27 for measuring indoor temperature, as well as of outdoor sensors 37 for outdoor temperature, 33 for outdoor concentration of nitric oxides NOₓ and 34 for outdoor monoxide concentration.

The device 1 can be designed in several ways. One embodiment is shown in Figs. 2, 3, 7 - 9. Its front with the indicating arrangements 6 is explained above in detail as represented in Fig. 2.

Fig. 3 shows a lateral view of device 1 with a number of ventilation slots 11 for allowing circulation of air within the device 1 and allowing in particular access of the air to the sensors 21-26.

Fig. 7 shows a bottom view of device 1 with four cavities 12 for receiving batteries and a DC power jack 13 for attachment to an electric source 7.

Fig. 8 shows a top view of device 1 with ventilation slots 11 and a number of function keys which are explained from right to left:
button 14 (with the "light" symbol); if it is pressed once backlight is turned on for 5 seconds;
button 18 for adjusting the air quality level by the user;
button 15 (with the "up" symbol) is used for toggling between maximum temperature/humidity memory and the current readings; Up setting when in "User define air quality mode";
button 16 (with the "down" symbol) is used for toggling between minimum temperature/humidity memory and the current readings; Down setting when in "User define air quality mode";
button 17 (with the "clear" symbol) is used for clearing the maximum and minimum temperature/humidity memories;
button 10 (with the "1/15" symbol) is used for toggling between normal and fast sensing mode (as described above); if pressed and hold for 2 seconds the "User defined air quality mode" is entered; during the "User Adjustment Mode", the current Adjustment Setting will be flashing to indicate it is the User Adjustment Mode.

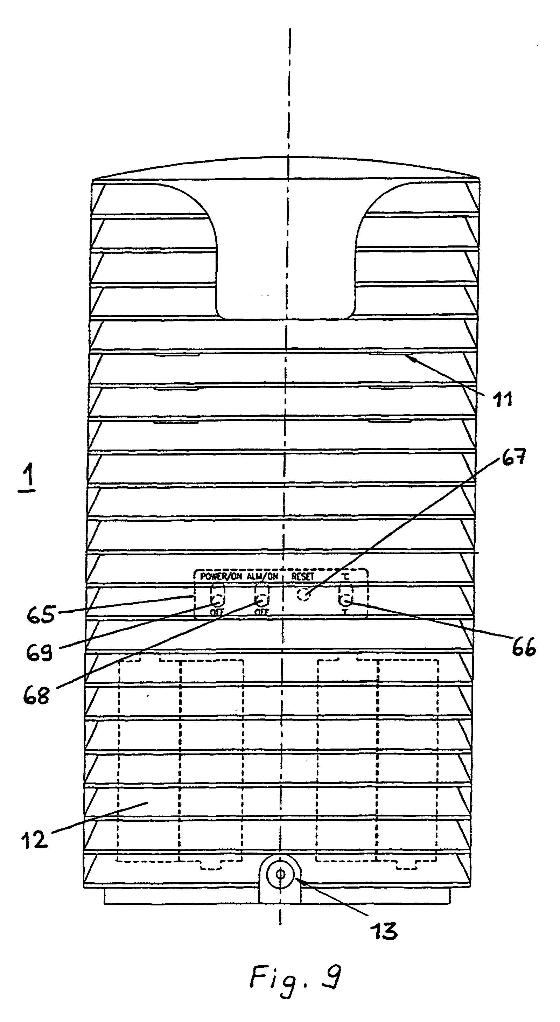

Fig. 9 shows a back view of the device 1 with the DC power jack 13 for attachment to an electric source 7, the four cavities 12 for receiving batteries, and a number of ventilation slots 11. On the rear key panel 65 four buttons can be distinguished from right to left:
button 66 (with the "°C/°F" symbol) for user selection of temperature units either in °C or °F;
button 67 (with "reset" designation) for resetting the device 1 if in an abnormal condition;
button 68 (with "ALM/ON OFF" designation) for enabling or disenabling the warning alarm for air quality;
button 69 (with "POWER ON/OFF" designation) is the power switch for the device 1; the user can turn off the device 1 to save battery life.

Hereinafter the Operation of the device is shortly described:
1.0 Reset /Power Reset
   1.1. If button 67 is pushed all segments of the LCD displays in the indicating arrangements 6 will be turned on and a beep sound will be given out for 3 seconds; the device 1 will enter the "Operation" mode;
   1.2. The sampling rate will be set at 1 per each minute and a 15 minute bar chart will be shown in display 63;
   1.3. The indicator for the User defined Level Adjustment in display 64 at far left will show only the default level; all arrows will be turned off;
   1.4. The readings other than -OH will be blanked until there are sufficient data to show the reading.
2.0. Operation Mode
   2.1. The current indoor temperature and humidity will be shown on the LCD;
   2.2. User can press the UP key to toggle the Maximum temperature/humidity and the current reading;
   2.3. User can press the DOWN key to Minimum Temperature/Humidity reading and the current reading;
   2.4. When it is not showing the current reading, the current readings will be shown automatically after 5 seconds;
   2.5. User can press the CLEAR key to replace the Maximum and Minimum temperature/humidity with the Current reading;
   2.6. If the RATE key is pressed the Sampling Rate and the Time Scale is changed of the Bar Graph. The Sampling rate will change from 1 sample per 15 minutes to 1 sample per minute;
   2.7. when the sampling Rate is 1 per 15 minutes, a 6 hour bar chart will be shown; if the sampling rate is 1 per each minute, a 15 minute bar chart will be shown;
   2.8. when the sampling rate is changed to 1 per each minute , the 15 minute bar chart will have nothing to show. It will take 15 minutes to fill up the whole bar history;
   2.9. 1 minute after the bar chart is filled up, the sampling rate will switch to 1 per 15 minutes automatically.
   2.10. During the 15 bar chart is being filled up, user can press the RATE key to toggle between the 6 hour bar chart and the 15 minute bar chart. If user switches back to the 15 minute bar chart within 2 minutes, the filling up of the 15 minute bar chart will be continued. Otherwise, the filling up of the 15 minute bar chart will start from the beginning as under point 2.8.;
   2.11. For entering the "User Adjustment Mode" the RATE key has to be pressed and hold for 2 seconds;
   2.12. When the 6 hour bar chart is completed, the bars will be displayed one by one for each second (total of 9 seconds for the whole chart). After all bars are shown, the bar chart will keep steady for 6 seconds, then the bar chart will be cleared (for 1 second) and repeat the same steps again.
3. User Adjustment Mode
   3.1. User can press and hold the RATE key for 2 seconds to enter the User Adjustment Mode.
   3.2. During the User Adjustment Mode, the current User Adjustment Setting will be flashing in display 64 to indicate it is the User Adjustment Mode;
   3.3. If UP and DOWN keys are pressed the adjustment level is changed. By pressing the UP key, the user can make the device more sensitive to the VOC gases. By pressing the DOWN key, the user can make the device less sensitive to the VOC gases. The User Adjustment Indicators in display 64 will show the level of sensitivity by the arrows pointing up or down.
   3.4. If the RATE key is pressed the User Adjustment Mode is left and function of the device is returned to the normal operation.
   3.5. There are six User Adjustment Levels: 3 arrows up, 2 arrows up, 1 arrow up, no adjustment, one arrow down, two arrows down, three arrows down;
   3.6. The level change of the Adjustment Level will affect the bar chart. The number of level changes (arrows down/up) will cause the bar display to move up/down with the same number of steps, e.g. if the bar reading is FAIR 3 for no adjustment, then the change of Adjustment Level to on level down will make the Bar readings become FAIR 2 (also one level down);
   3.7. The User Adjustment Setting does not effect the Warning Alarm function.
4.0 Backlight
   4.1. The User can press the LIGHT key to enable the Backlight function;
   4.2. the Backlight will be turned on until there is no key press or key hold within 5 seconds.
5.0. Warning Alarm
   5.1. When the Air Quality Reading is beyond the Warning level, a Warning Alarm will be given;
   5.2. The Warning level is set at a concentration of 90 ppm of acetone or any other value recommended by governmental or scientific authorities;
   5.3. User can use the Alarm Sound On/Off Slide Switch to disable the Alarm Sound or press any key to stop the Alarm Sound;
   5.4. During the Warning Alarm is giving out, the "FAN" symbol and the "WINDOW" symbol will be altering to show a "Powered Fan" and an "Opening Window" in display 64.
   5.5. The Alarm will keep going for 6 hours until the Air Quality is improved;
   5.6. The Alarm pattern is:
   10 beep tones (one per second) within 20 seconds for the First minute. Then 5 beep tones within 10 seconds for every minute.

## Claims

1. Device (1) for monitoring the air quality comprising
A) a sensor arrangement (2) with at least one sensor (21-26) for measuring at least one chemical gas or a gas mixture which plays an influential part in air quality,
B) a computer (3),
C) an electronic clock (4),
D) a memory (5); and
E) indicating arrangements (6)
**characterized in that**
F) said measuring at least one chemical gas or a gas mixture by said sensor arrangement (2) is performable automatically at given intervals by means of said computer (3) and electronic clock (4); and
G) said indicating arrangements (6) allow by means of said computer (3), electronic clock (4) and memory (5) to simultaneously indicate current measured values and ones which have been measured with the sensor arrangement (2) within a given past period of time at said given intervals thereby indicating a history of the measured values.

2. Device (1) according to claim 1, **characterized in that** a trend of said history of the measured values can be displayed at the indicating arrangements (6).

3. Device (1) according to claim 1 or 2, **characterized in that** the indication of air quality comfort and its numerical or symbolic display on the indicating arrangements (6) can be adjusted to individual perception by lowering or increasing threshold-values triggering changes in the display.

4. Device (1) according to one of the claims 1 to 3, **characterized in that** the length of said given intervals are 1 to 10 % of the length of said given past period of time.

5. Device (1) according to one of the claims 1 to 4, **characterized in that** intervals are intervals of 0,5 to 2,0 minutes, preferably of 0,9 to 1,1 minutes, for measuring short term events.

6. Device (1) according to one of the claims 1 to 4, **characterized in that** intervals are intervals of 10 to 20 minutes, preferably of 14 to 16 minutes for measuring medium term events.

7. Device (1) according to one of the claims 1 to 6, **characterized in that** said measured values and/or said measured values trends are indicated in digital form, preferably by means of a bar chart display at the indicating arrangements (6).

8. Device (1) according to one of the claims 1 to 7, **characterized in that** part of the computer (3) is set up as an algorithm producer which deduces symbols for the level of air quality from measured values and/or measured values trends fed into the computer (3) and that said symbols for the level of air quality can be displayed at the indicating arrangements (6).

9. Device (1) according to one of the claims 1 to 8, **characterized in that** said sensor arrangement (2) comprises a sensor (21) for measuring volatile organic compounds VOC.

10. Device (1) according to one of the claims 1 to 9, **characterized in that** said sensor arrangement (2) comprises a sensor (22) for measuring carbon dioxide CO₂.

11. Device (1) according to one of the claims 1 to 10, **characterized in that** said sensor arrangement (2) comprises a sensor (23) for measuring nitric oxides NOₓ.

12. Device (1) according to one of the claims 1 to 11, **characterized in that** said sensor arrangement (2) comprises a sensor (24) for measuring carbon monoxide CO.

13. Device (1) according to one of the claims 1 to 12, **characterized in that** said sensor arrangement (2) comprises a sensor (25) for measuring reducing gases.

14. Device (1) according to one of the claims 1 to 13, **characterized in that** said sensor arrangement (2) comprises a sensor (26) for measuring oxidizing gases.

15. Device (1) according to one of the claims 1 to 14,
**characterized in that** said sensor arrangement (2) comprises additionally a temperature sensor (27).

16. Device (1) according to one of the claims 1 to 15, **characterized in that** said sensor arrangement (2) comprises additionally a barometric pressure sensor (28).

17. Device (1) according to one of the claims 1 to 16, **characterized in that** said sensor arrangement (2) comprises additionally a relative air humidity sensor (29).

18. Device (1) according to one of the claims 1 to 17, **characterized in that** said sensor arrangement (2) comprises two sensors (21-29) for each parameter to be measured, one sensor (21-29) for indoor measurement and one sensor (31-39) for the same parameter for outdoor measurement.

19. Device (1) according to claim 18, **characterized in that** said the device (1) allows indication, at the indicating arrangements (6), whether comfortable and/or safe conditions prevail indoor in the room in which the device (1) is set up compared to the outdoor conditions.

20. Device (1) according to one of the claims 1 to 19, **characterized in that** the device generates by means of algorithms and displays on the indicating arrangements (6) symbols for recommended actions to be performed by the user of the device.

21. Device (1) according to one of the claims 1 to 20, **characterized in that** said sensor arrangement (2) comprises three or more sensors (21-29) for each parameter to be measured, one or more sensors (21-29) for indoor measurement and one or more sensors (31-39) for the same parameter for outdoor measurement.

## Patentansprüche

1. Gerät (1) zur Überwachung der Luftqualität umfasst:
A) eine Sensorenanordnung (2) mit wenigstens einem Sensor (21-26) für die Messung von wenigstens einem chemischen Gas oder einer Gasmischung, die eine einflussreiche Rolle bei der Luftqualität spielt,
B) ein Rechner (3),
C) eine elektronische Uhr (4),
D) ein Speicher (5); und
E) Anzeigeanordnungen (6) die dadurch charakterisiert sind, dass
F) die besagte Messung von wenigstens einem chemischen Gas oder einer Gasmischung durch die genannte Sensoranordnung (2) automatisch in vorgegebenen Intervallen mit Hilfe des genannten Rechners (3) und der elektronischen Uhr (4) durchgeführt werden kann; und
G) dass die genannten Anzeigeanordnungen (6) mittels des genannten Rechners (3), der elektronischen Uhr (4) und des Speichers (5) in der Lage sind, gleichzeitig aktuelle Messwerte und solche anzugeben, die mit der genannten Sensoranordnung (2) innerhalb einer vorgegebenen, vergangenen Zeitspanne in den genannten Zeitintervallen gemessen wurden und so eine Historie der Messwerte anzeigen.

2. Gerät (1) entsprechend Anspruch 1, charakterisiert dadurch, dass ein Trend der genannten Historie der Messwerte über die Anzeigeanordnungen (6) angezeigt werden kann.

3. Gerät (1) entsprechend Anspruch 1 oder 2, charakterisiert dadurch, dass die Anzeige der Luftqualität und die numerische oder symbolische Anzeige auf den Anzeigeanordnungen (6) durch Senken oder Anheben der Schwellenwerte, die die Veränderungen in der Anzeige auslösen, an die individuelle Wahrnehmung angepasst werden kann.

4. Gerät (1) entsprechend einem der Ansprüche 1 bis /Zahl unleserlich/, charakterisiert dadurch, dass die Länge der genannten, vorgegebenen Intervalle von 1% bis 10 % der Länge der genannten vergangenen Zeitspanne umfasst.

5. Gerät (1) entsprechend einem der Ansprüche 1 bis 4, charakterisiert dadurch, dass die Intervalle intervalle von 0,5 bis 2,0 Minuten sind, vorzugsweise 0,9 bis 1,1 Minuten für die Messung kurzfristiger Ereignisse.

6. Gerät (1) entsprechend einem der Ansprüche 1 bis 4, charakterisiert dadurch, dass die Intervalle Invervalle von 10 bis 20 Minuten sind, vorzugsweise 14 bis 16 Minuten, für die Messung mittelfristiger Ereignisse.

7. Gerät (1) entsprechend einem der Ansprüche 1 bis 6, charakterisiert dadurch, dass die genannten Messwerte und/oder die genannten Trends der Messwerte in digitaler Form angegeben werden, vorzugsweise mittels einer Säulendiagrammanzeige über die Anzeigeanordnungen (6).

8. Gerät (1) entsprechend einem der Ansprüche 1 bis 7, charakterisiert dadurch, dass ein Teil des Rechners (3) als Produzent von Algorithmen eingesetzt wird und Symbole für das Niveau der Luftqualität aus den Messwerten und/oder den Trends der Messwerte in den Rechner (3) eingegeben werden und dass die genannten Symbole für das Niveau der Luftqualität über die Anzeigeanordnungen (6) ausgegeben werden können.

9. Gerät (1) entsprechend einem der Ansprüche 1 bis 8, charakterisiert dadurch, dass die genannte Sensoranordnung (2) einen Sensor (21) zur Messung von flüchtigen organischen Verbindung FOV umfasst.

10. Gerät (1) entsprechend einem der Ansprüche 1 bis 9, charakterisiert dadurch, dass die genannte Sensoranordnung (2) einen Sensor (22) zur Messung von Kohlendioxid CO₂ umfasst.

11. Gerät (1) entsprechend einem der Ansprüche 1 bis 10, charakterisiert dadurch, dass die genannte Sensoranordnung (2) einen Sensor (23) zur Messung von Stickoxiden NO_{X} umfasst.

12. Gerät (1) entsprechend einem der Ansprüche 1 bis 11, charakterisiert dadurch, dass die genannte Sensoranordnung (2) einen Sensor (24) zur Messung von Kohlenmonoxid CO umfasst.

13. Gerät (1) entsprechend einem der Ansprüche 1 bis 12, charakterisiert dadurch, dass die genannte Sensoranordnung (2) einen Sensor (25) zur Messung von reduzierenden Gasen umfasst.

14. Gerät (1) entsprechend einem der Ansprüche 1 bis 13, charakterisiert dadurch, dass die genannte Sensoranordnung (2) einen Sensor (26) zur Messung von oxidierenden Gasen umfasst.

15. Gerät (1) entsprechend einem der Ansprüche 1 bis 14, charakterisiert dadurch, dass die genannte Sensoranordnung (2) einen zusätzlichen Temperatursensor (27) umfasst.

16. Gerät (1) entsprechend einem der Ansprüche 1 bis 15, charakterisiert dadurch, dass die genannte Sensoranordnung (2) einen zusätzlichen Sensor zur barometrischen Druckmessung (28) umfasst.

17. Gerät (1) entsprechend einem der Ansprüche 1 bis 16, charakterisiert dadurch, dass die genannte Sensoranordnung (2) einen zusätzlichen Sensor zur Messung der relativen Luftfeuchtigkeit (29) umfasst.

18. Gerät (1) entsprechend einem der Ansprüche 1 bis 17, charakterisiert dadurch, dass die genannte Sensoranordnung (2) zwei Sensoren (21-29) für jeden zu messenden Parameter umfasst, ein Sensor (21-29) für Innenraummessung und ein Sensor (31-39) für die gleichen Parameter für Außenmessung.

19. Gerät (1) entsprechend Anspruch 18, charakterisiert dadurch, dass das genannte Gerät (1) an den Anzeigeordnungen (6) die Anzeige ermöglicht, ob in dem Raum, in dem das Gerät (1) installiert ist, im Vergleich zu den Außenbedingungen bequeme und/oder sichere Bedingungen vorherrschen.

20. Gerät (1) entsprechend einem der Ansprüche 1 bis 19, charakterisiert dadurch, dass das Gerät durch Algorithmen und Anzeigen auf den Anzeigeanordnungen (6) Symbole für empfohlene Handlungen erzeugt, die von Anwender des Geräts vorgenommen werden sollen.

21. Gerät (1) entsprechend einem der Ansprüche 1 bis 20, charakterisiert dadurch, dass die genannte Sensoranordnung (2) drei oder mehr Sensoren (21-29) für jeden zu messenden Parameter einen oder mehr Sensoren (21-29) für Innenraummessung und einen oder mehr Sensoren (31-39) für die gleichen Parameter für Außenmessung umfasst.

## Revendications

1. Dispositif (1) de contrôle de la qualité de l'air, comprenant
A) un ensemble de capteurs (2) avec au moins un capteur (21-26) pour mesurer au moins un gaz chimique ou un mélange gazeux qui joue un rôle déterminant dans la qualité de l'air,
B) un ordinateur (3),
C) une horloge électronique (4),
D) une mémoire (5); et
E) des éléments indicateurs (6)
**caractérisé en ce que**
F) ladite mesure d'au moins un gaz chimique ou un mélange gazeux par ledit ensemble de capteurs (2) est réalisable automatiquement à intervalles donnés, au moyen dudit ordinateur (3) et de ladite horloge électronique (4); et **en ce que**
G) lesdits éléments indicateurs (6) permettent, à l'aide desdits ordinateur (3), horloge électronique (4) et mémoire (5), d'indiquer simultanément les valeurs mesurées actuelles et celles qui ont été mesurées par l'ensemble de capteurs (2) à intervalles donnés au cours d'une période passée donnée, fournissant ainsi un historique des valeurs mesurées.

2. Dispositif (1) selon la revendication 1, **caractérisé en ce qu'**une tendance dudit historique des valeurs mesurées peut s'afficher sur les éléments indicateurs (6).

3. Dispositif (1) selon la revendication 1 ou 2, **caractérisé en ce que** l'indication du degré de qualité de l'air et son affichage numérique ou symbolique sur les éléments indicateurs (6) peuvent être adaptés à la perception individuelle en abaissant ou en élevant les valeurs seuils, déclenchant des modifications de l'affichage.

4. Dispositif (1) selon une des revendications 1 à 3, **caractérisé en ce que** la durée desdits intervalles donnés se situe entre 1 et 10% de la durée de ladite période passée donnée.

5. Dispositif (1) selon une des revendications 1 à 4, **caractérisé en ce que** les intervalles sont des intervalles de 0,5 à 2,0 minutes, de préférence de 0,9 à 1,1 minute, pour la mesure d'événements à court terme.

6. Dispositif (1) selon une des revendications 1 à 4, **caractérisé en ce que** les intervalles sont des intervalles de 10 à 20 minutes, de préférence de 14 à 16 minutes, pour la mesure d'événements à moyen terme.

7. Dispositif (1) selon une des revendications 1 à 6, **caractérisé en ce que** lesdites valeurs mesurées et/ou lesdites tendances des valeurs mesurées s'affichent sous forme numérique sur les éléments indicateurs (6), de préférence au moyen d'un graphique en bâtons.

8. Dispositif (1) selon une des revendications 1 à 7, **caractérisé en ce qu'**une partie de l'ordinateur (3) est réglée comme émetteur d'algorithmes qui déduit les symboles du niveau de qualité de l'air à partir des valeurs mesurées et/ou des tendances des valeurs mesurées fournies à l'ordinateur (3), et **en ce que** lesdits symboles du niveau de qualité de l'air peuvent s'afficher sur les éléments indicateurs (6).

9. Dispositif (1) selon une des revendications 1 à 8, **caractérisé en ce que** ledit ensemble de capteurs (2) comprend un capteur (21) pour mesurer les composés organiques volatils COV.

10. Dispositif (1) selon une des revendications 1 à 9, **caractérisé en ce que** ledit ensemble de capteurs (2) comprend un capteur (22) pour mesurer le dioxyde de carbone CO₂.

11. Dispositif (1) selon une des revendications 1 à 10, **caractérisé en ce que** ledit ensemble de capteurs (2) comprend un capteur (23) pour mesurer les oxydes nitriques NOₓ.

12. Dispositif (1) selon une des revendications 1 à 11, **caractérisé en ce que** ledit ensemble de capteurs (2) comprend un capteur (24) pour mesurer le monoxyde de carbone CO.

13. Dispositif (1) selon une des revendications 1 à 12, **caractérisé en ce que** ledit ensemble de capteurs (2) comprend un capteur (25) pour mesurer les gaz réducteurs.

14. Dispositif (1) selon une des revendications 1 à 13, **caractérisé en ce que** ledit ensemble de capteurs (2) comprend un capteur (26) pour mesurer les gaz oxydants.

15. Dispositif (1) selon une des revendications 1 à 14, **caractérisé en ce que** ledit ensemble de capteurs (2) comprend en plus un capteur de température (27).

16. Dispositif (1) selon une des revendications 1 à 15, **caractérisé en ce que** ledit ensemble de capteurs (2) comprend en plus un capteur de hauteur barométrique (28).

17. Dispositif (1) selon une des revendications 1 à 16, **caractérisé en ce que** ledit ensemble de capteurs (2) comprend en plus un capteur d'humidité relative de l'air (29).

18. Dispositif (1) selon une des revendications 1 à 17, **caractérisé en ce que** ledit ensemble de capteurs (2) comprend deux capteurs (21-29) pour chaque paramètre devant être mesuré, un capteur (21-29) pour la mesure à l'intérieur et un capteur (31-39) pour la mesure du même paramètre à l'extérieur.

19. Dispositif (1) selon la revendication 18, **caractérisé en ce que** ledit dispositif (1) permet d'indiquer, sur les éléments indicateurs (6), si les conditions qui règnent dans la pièce où est installé le dispositif (1) sont agréables et/ou saines par rapport aux conditions régnant à l'extérieur.

20. Dispositif (1) selon une des revendications 1 à 19, **caractérisé en ce que** le dispositif crée, à l'aide d'algorithmes et d'affichages sur les éléments indicateurs (6), des symboles qui conseillent les mesures à prendre par l'utilisateur du dispositif.

21. Dispositif (1) selon une des revendications 1 à 20, **caractérisé en ce que** ledit ensemble de capteurs (2) comprend trois capteurs (21-29) ou plus pour chaque paramètre devant être mesuré, un capteur (21-29) ou plus pour la mesure à l'intérieur et un capteur (31-39) ou plus pour la mesure du même paramètre à l'extérieur.
